# FASCICULE DE BREVET EUROPEEN

(11) **EP 0 724 859 B1**
(45) Date de publication et mention de la délivrance du brevet: **12.11.1997**
(21) Numéro de dépôt: 96101177.2
(22) Date de dépôt: 29.01.1996
(51) Int. Cl.: A61B 5/00

(54) **Système individuel de mesure, de traitement et de transmission de paramètres essentiellement physiologiques**
Einzelne Anordnung zur Messung, Verarbeitung und Übertragung von im wesentlichen physiologischen Parametern
Personal device for measurement, processing and transmission of substantially physiological data

(30) Priorité: 04.02.1995 CH 295/95
(43) Date de publication de la demande: 07.08.1996
(73) Titulaire: Baumann & Haldi S.A., 2114 Fleurier (CH)
(72) Inventeur: Sarbach, Pierre, CH-1040 Echallens (CH)
(74) Mandataire: Thérond, Gérard Raymond

(56) Documents cités:
- WO-A-91/18550
- GB-A- 2 259 772
- US-A- 3 972 320
- PROCEEDINGS OF THE 7TH ANNUAL CONFERENCE OF THE IEEE/EMB SOCIETY, 30 Septembre 1985, CHICAGO (US), pages 1205-1210, XP000572257 J.H. SCHILD ET AL.: "A Low-Power Multichannel Biotelemeter"

## Description

La présente invention a pour objet un système individuel de mesure de traitement et de transmission de paramètres essentiellement physiologiques.

Il est en effet de plus en plus important dans le traitement de nombreuses maladies, comme dans la pratique de plusieurs sports, de connaître avec précision certains paramètres physiologiques d'un individu, comme le rythme cardiaque, la pression, la teneur en sucre du sang, ainsi que des paramètres liés à l'activité sportive comme la vitesse ou la puissance développée par l'usager. A cette fin, de nombreux types de capteurs légers et faciles à fixer sur le porteur ont été développés ces dernières années, allant de simples contacts électriques pour relever un électrocardiogramme (ECG), à des capteurs complexes directement incorporés par exemple dans des puces silicium.

La plupart de ces capteurs délivrent des signaux électriques en rapport avec le(s) paramètre(s) à mesurer et porteurs de nombreuses informations utiles. Par exemple, pour un électrocardiogramme, on aura des impulsions caractéristiques dont la fréquence est l'image directe du rythme cardiaque. Cependant une analyse plus approfondie de la forme de ces impulsions pourrait donner des informations supplémentaires sur certains disfonctionnements et pourrait ainsi permettre de prévenir certains accidents cardiaques.

Le traitement des signaux délivrés par les capteurs ne pose pas de problème dans le cas où l'individu peut être relié à une installation fixe, par exemple lorsque la personne est alitée, ou n'est reliée que pendant une durée relativement courte à une installation de mesure fixe. Une telle conception correspond par exemple au dispositif décrit dans le document GB-A-2 259 772, dans lequel des appareils de détection portés par plusieurs usagers transmettent par ondes radio des données biologiques à une unité centrale. Cette solution est également celle retenue dans les travaux publiés par J.H. Schild et al. (Proceedings of the 7th annual conference of the IEEE/EMB society, 30 September 1995, Chicago, pages 1205-1210). Il en va tout autrement si l'on veut avoir un contrôle de longue durée et/ou si la personne doit garder toute sa mobilité, tout en pouvant accéder facilement à la lecture des paramètres désirés. A l'heure actuelle, il est possible de réaliser des dispositifs de traitement de signaux petits et autonomes pouvant être fixés sur l'individu, par exemple directement sur l'abdomen au moyen d'une ceinture ou de sangles. Il est aisé de comprendre dans le cas cité ci-dessus que les moyens d'affichage des paramètres, pour être facilement lisibles, doivent être séparés du système de traitement des signaux. Une liaison par fil est toujours délicate dans la mesure où ce fil peut facilement se casser lorsque la personne se déplace.

Un exemple intéressant de liaison sans fil est donné par certains systèmes de lecture du rythme cardiaque utilisé pour l'entraînement de sportifs de haut niveau. Ces systèmes comprennent une ceinture comportant des capteurs de signal ECG et un dispositif simple de mise en forme de ce signal générant des impulsions à la fréquence de battement du coeur. Ces impulsions sont ensuite transmises sans fil par modulation tout ou rien d'un signal radio vers un dispositif de réception, de traitement et de mesure de ces signaux, permettant de calculer et d'afficher la fréquence cardiaque. Le dispositif de réception et d'affichage peut par exemple être incorporé dans une montre fixée au poignet, ou placée sur le guidon d'une bicyclette, de la manière la plus appropriée selon le genre d'utilisation. Il peut alors être combiné avec d'autres fonctions, particulièrement des fonctions heure ou chrono. Un dispositif de ce type est par exemple décrit dans le document FR 2 685 189. Le premier défaut important des systèmes connus réside dans le fait que la mesure est effectuée au niveau du dispositif de réception. Ainsi toute perturbation de la liaison radio peut introduire des erreurs de mesure et un affichage erroné de la fréquence cardiaque. Le second défaut important est la limitation d'un tel système. En effet dans sa conception actuelle, il ne permet que la mesure du rythme cardiaque puisqu'il se contente de transmettre des impulsions à ce rythme. Hors, comme nous l'avons vu plus haut, certaines applications nécessitent une analyse plus poussée du signal ECG, voir l'analyse de paramètres donnés par d'autres types de capteurs. Un dernier point important consiste à réduire au maximum la consommation d'énergie, particulièrement au niveau du dispositif de réception et d'affichage, lequel est le plus souvent alimenté par batterie et doit présenter une autonomie la plus grande possible, surtout s'il est également utilisé comme montre comme dit plus haut.

La présente invention apporte des solutions originales et performantes allant dans ce sens. Elle concerne un système de mesure et d'affichage d'au moins un paramètre essentiellement physiologique au moyen d'au moins un capteur porté par un individu, ledit capteur délivrant des signaux représentatifs dudit paramètre, comprenant d'une part un dispositif autonome de traitement solidaire dudit capteur et pourvu de moyens de mesure, de stockage, d'identification et de codage desdits signaux, lesdits moyens de codage étant agencés de manière à générer aux bornes d'une bobine émettrice des séquences binaires de signaux radio, une desdites séquences constituant une adresse permettant une identification du dispositif de traitement émetteur, les autres séquences étant représentatives de chaque catégorie de mesure et de leur résultat, et d'autre part un dispositif d'affichage séparé et relié au dispositif de traitement par des moyens de communication sans fil, ledit dispositif séparé d'affichage comportant une bobine réceptrice, des moyens d'amplification et de mise en forme des signaux aux bornes de ladite bobine réceptrice et des moyens de décodage de ces signaux caractérisé en ce que les moyens de décodage du dispositif d'affichage comportent un générateur de séquences agencé de manière à ouvrir des fenêtres de détection synchrones avec les trains d'impulsions émis par le dispositif de traitement, et à restituer la suite binaire de 1 et de 0 représentative des informations transmise en fonction de la répartition desdites impulsions dans lesdites fenêtres de détection et en ce que les informations transmises par ledit dispositif de traitement sont restituées et affichées seulement si une adresse déterminée a préalablement été identifiée.
- La figure 1 représente de manière schématique à titre d'exemple un dispositif de mesure porté par l'usager et formant un des éléments constitutifs du système selon l'invention;
- la figure 2 représente de manière schématique à titre d'exemple un dispositif d'affichage du système selon l'invention;
- la figure 3 représente un autre mode de réalisation du dispositif de mesure porté par l'usager;
- la figure 4 représente à titre d'exemple le schéma bloc d'un dispositif de traitement selon l'invention;
- la figure 5 représente la forme caractéristique des signaux à certains points du schéma de la figure 4, et
- la figure 6 représente à titre d'exemple le schéma bloc des moyens de décodage du dispositif d'affichage permettant de restituer les informations envoyées par le dispositif de traitement.

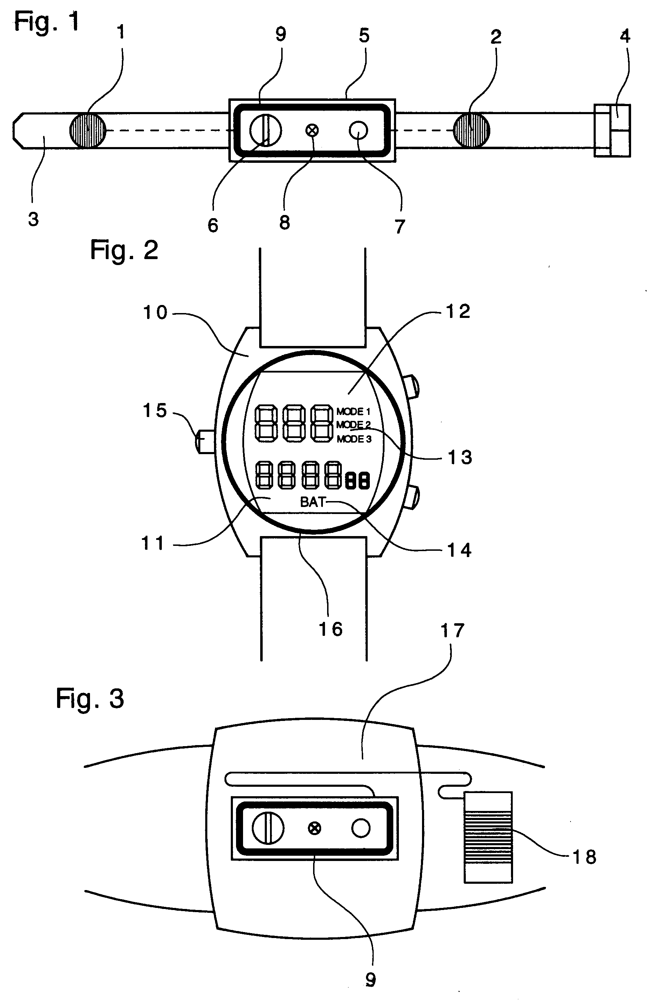

La figure 1 représente de manière schématique à titre d'exemple un dispositif de mesure porté par l'usager et formant un des éléments constitutifs du système selon l'invention. Par exemple le ou les capteurs 1, 2 peuvent être montes sur une ceinture 3 pouvant être serrée autour de la taille soit au moyen d'une boucle de fixation 4, soit par une fixation de type Velcro.

Dans le cas représenté, les capteurs 1 et 2 sont de simples contacts électriques par exemple en caoutchouc conducteur permettant de détecter le signal ECG. Ces capteurs sont reliés directement au dispositif autonome de traitement des signaux 5, fixé également sur la ceinture. Ce dispositif de traitement 5 comporte typiquement un circuit électronique qui sera décrit en détail plus loin, une batterie avec un système d'accès 6 permettant un changement facile de celui-ci, un poussoir ou un interrupteur 7 permettant de mettre en service ou de réinitialiser le dispositif, et des moyens d'affichage, pouvant être comme ici une simple diode LED 8, permettant de contrôler le bon fonctionnement du dispositif. Enfin, le dispositif comporte une bobine émettrice 9 permettant d'assurer la liaison radio avec les moyens d'affichage.

Ces moyens d'affichage sont représentés dans notre exemple à la figure 2 sous forme d'une montre digitale 10 comportant un affichage classique des heures, minutes et secondes 11, et un affichage spécial 12 des paramètres mesurés par le dispositif de traitement 5. L'affichage peut également comporter des flags et des symboles correspondant aux paramètres à afficher 13 ou à certaines circonstances spéciales 14, par exemple la fin de vie des batteries.

La montre comporte également des poussoirs 15 permettant d'appeler les différentes fonctions et, le cas échéant de les corriger, et une bobine réceptrice 16 qui permet de recevoir les signaux radio du dispositif de traitement 5. La montre peut également comporter un buzzer permettant d'attirer l'attention du porteur en cas de besoin. Cette fonction n'est pas représentée dans la mesure où elle est parfaitement connue des gens de métier.

Notons que les moyens d'affichage peuvent également être agencés de manière à pouvoir être fixés sur un autre support que la personne même, par exemple au guidon d'une bicyclette dans le cas d'un cycliste, ou à proximité de la tête d'un lit dans le cas où il s'agit d'une personne alitée. Dans ce dernier cas, les moyens d'affichage peuvent être associés à un système d'assistance ou d'alarme permettant d'attirer l'attention du personnel en cas de problème. Il s'agit là toutefois d'applications particulières qui ne modifient pas fondamentalement le fonctionnement du système.

Le dispositif de traitement 5 peut également être fixé d'autres manières, par exemple, comme représenté à la figure 3, au moyen d'un brassard 17 sur l'arrière bras, et être associé à d'autres types de capteurs fixés à proximité, par exemple un capteur de température 21. D'autres capteurs, non assujettis à l'usager, peuvent être utilisés, tel qu'un capteur de vitesse ou un capteur de puissance développée par l'usager. Il existe également actuellement des capteurs capables de mesurer certains paramètres du sang, par exemple le taux de sucre. La mesure de ce paramètre est essentielle dans le traitement du diabète et il serait possible en associant ce type de capteurs au système selon l'invention d'afficher cette valeur et de déterminer quand le porteur a besoin d'insuline et en quelle quantité.

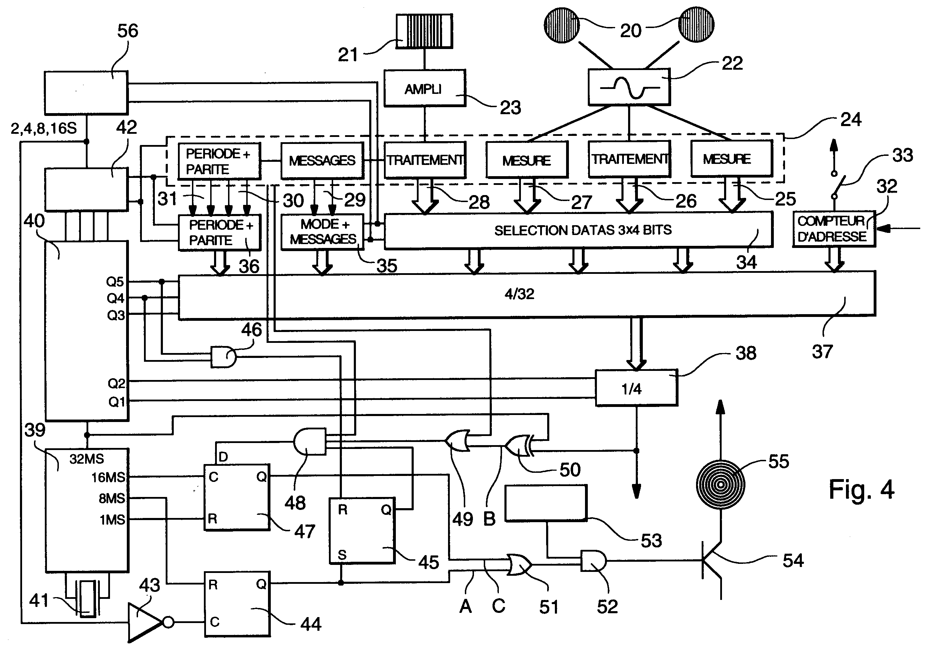

La figure 4 représente à titre d'exemple le schéma bloc d'un dispositif de traitement selon l'invention. Ce dispositif de traitement peut utiliser plusieurs types de capteurs, par exemple un capteur de signal ECG 20 et un capteur de température 21. Chacun de ces capteurs est relié à un circuit d'amplification et de mise en forme du signal 22 et 23, reliés à leur tour aux moyens de traitement et de mesure de ces signaux 24. Ces moyens 24 délivrent des informations binaires pouvant être aussi bien en rapport avec les résultats de ces mesures, que leur identification ou leur classement. Ainsi, à partir d'un seul capteur, par exemple le capteur du signal ECG 20, les moyens de traitement 24 pourraient déterminer plusieurs paramètres. Premièrement, la fréquence cardiaque instantanée 25, la moyenne de celle-ci sur par exemple 100 battements 26, et les variations de celle-ci exprimée à la hausse ou à la baisse du pourcentage 27. On a ainsi trois paramètres distincts déterminés à partir d'un même capteur. On peut également avoir un ou plusieurs paramètres déterminés à partir d'un ou plusieurs capteurs supplémentaires, typiquement le capteur 21 relié à l'amplificateur 23 et déterminant le paramètre 28. Les paramètres 25, 26, 27 et 28 sont tous représentés sous forme d'une information binaire 3 * 4 bits, représentant quatre chiffres décimaux.

Les moyens de traitement 24 sont représentés ici de manière très schématique sous forme de logique câblée relativement simple. Dans la pratique, on utilise plutôt de la logique programmée gérée par microprocesseur. Il existe actuellement toute une gamme de microprocesseurs à très faible consommation capable non seulement d'effectuer les différentes opérations de mesure et de traitement des mesures mentionnées ci-dessus, mais également d'effectuer la classification de ces mesures et de générer des messages de contrôle et d'erreur. Dans notre exemple, ces messages d'erreur sont représentés par deux bits permettant quatre combinaisons, chaque combinaison correspondant à un message particulier, fin de vie de batterie, mauvais contact, etc. Les moyens de traitement peuvent également générer des bits de parité 30 permettant de contrôler la bonne conformité des messages reçus. Enfin, les moyens de traitement 24 peuvent être utilisés pour optimiser la consommation d'énergie du dispositif de manière à obtenir l'autonomie la plus élevée possible. Cette consommation est en grande partie déterminée par la consommation de l'émetteur radio. Cela peut être obtenu en adaptant les périodes d'enclenchement de cet émetteur aux stricts besoins nécessaires par des algorithmes ad hoc gérés par les moyens de traitement. Dans ce cas, il faut bien sûr indiquer aux moyens d'affichage la valeur de ces périodes d'enclenchement. Dans notre exemple, cette période est représentée par une information de deux bits 31 correspondant à des périodes d'émission 2, 4, 8 et 16s.

A partir des données et autres informations fournies par les moyens de traitement, il s'agit de créer un train d'impulsions de durée fixe à transmettre au dispositif d'affichage. Ce train d'impulsions est formé de la manière suivante :
- 1, 2, 3, 4: : adresse
- 5, 6, 7, 8: : chiffre 1
- 9, 10, 11, 12: : chiffre 2
- 13, 14, 15, 16: : chiffre 3
- 17, 18: : mode
- 19, 20: : messages
- 21, 22: : période
- 23, 24: : parité

Chaque train d'impulsions correspond donc à une information de 24 bits, soit six groupes de quatre bits. L'adresse permet d'identifier l'émetteur. En effet, il se peut, dans certains cas, que deux émetteurs se trouvent à proximité l'un de l'autre. Il est donc important que le récepteur puisse différencier ces deux émetteurs l'un de l'autre. Le premier groupe correspond à cette adresse qui est fixée de manière aléatoire par exemple par un compteur de quatre bits 32 en fonction de la durée de fermeture d'un interrupteur 33.

Les trois groupes suivants correspondent à la valeur du paramètre exprimée par trois chiffres. Pour éviter d'avoir des trains d'impulsions trop longs et de durée variable, on n'envoie la valeur que d'un seul paramètre par train d'impulsions. Les différents paramètres, quatre dans le sens d'impulsions. Les différents paramètres, quatre dans le cas décrit, sont sélectionnés à tour de rôle par les moyens de traitement 24 par l'intermédiaire d'un démultiplexeur 34.

Le groupe suivant est constitué de deux bits 29 correspondant au mode ou paramètre sélectionné. Il est en effet important que les moyens d'affichage sachent à quel paramètre correspondent les valeurs transmises par les moyens de traitement. Les deux bits suivants correspondent à des messages spéciaux, alarme, fin de vie de pile, etc. Ces quatre bits sont mixés par le circuit 35.

Le dernier groupe est constitué de deux bits définissant la période de transmission déterminée par les moyens de traitement 24. Et les deux derniers bits sont des bits de parité permettant de contrôler la qualité de la transmission. Ces quatre bits sont mixés par le circuit 36.

On a ainsi six groupes de quatre bits. Chacun de ces groupes de quatre bits est sélectionné à tour de rôle par un démultiplexeur 4/32 37, puis chaque bit du groupe est sélectionné à tour de rôle par un démultiplexeur 1/4 38, de façon à créer une information binaire série formé d'une suite de 24 bits.

Pour créer cette information binaire série, on dispose d'un générateur 39, 40 piloté par un quartz 41. La première partie du générateur de séquences 39 est un compteur binaire qui génère les différents signaux nécessaires à la formation des impulsions. La deuxième partie de ce générateur est un compteur binaire 40 qui génère les différents signaux nécessaires pour commander les démultiplexeurs 37 et 38 (sorties Q1, Q2, Q3, Q4 et Q5) de manière à former le train d'impulsions de 24 bits formant l'information à transmettre au dispositif d'affichage. Par ailleurs, ce compteur 40 fixe quatre périodes de répétition 2, 4, 8 et 16s des trains d'impulsions peuvent être choisis par le sélecteur 1/4 42 en fonction de la période déterminée par les moyens de traitement 24.

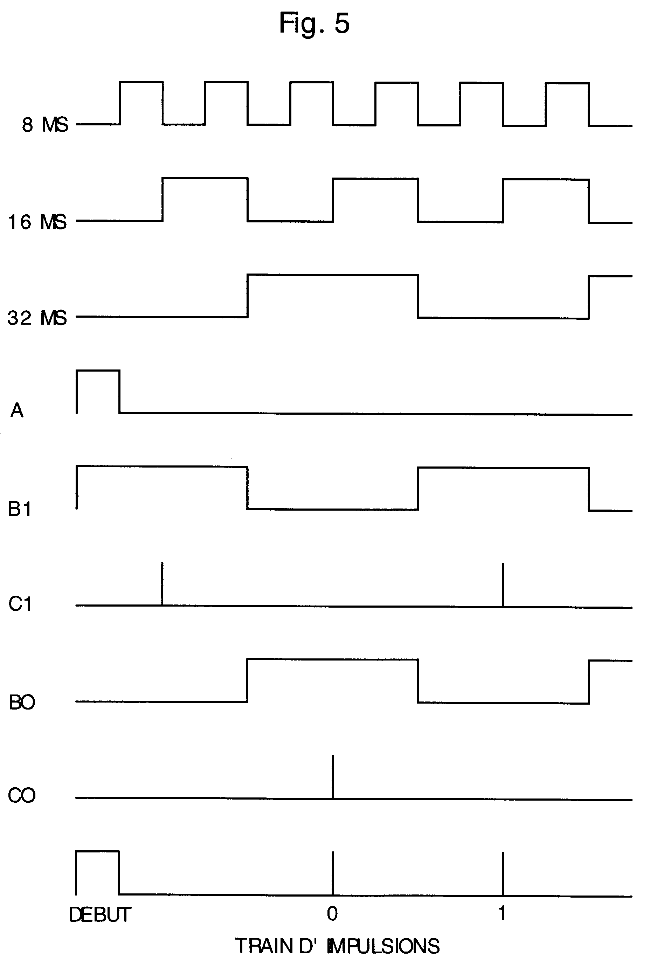

Le signal de sortie du sélecteur 42, est appliqué par l'intermédiaire d'un inverseur 43 à l'entrée d'horloge d'un FFd 44 dont l'entrée reset est reliée à la sortie 8ms du compteur 39. Ainsi, le FFd 44 va générer sur sa sortie Q une impulsion de 4 ms au début de chaque période de transmission. Cette sortie Q est branchée à l'entrée set d'un FFd 45 dont la sortie reset est reliée à la sortie d'une porte AND 46 dont les entrées sont reliées aux sorties Q4 et Q5 du compteur 40. Ainsi le FFd 45 va passer à 1 pendant 24 périodes d'entrée du compteur 40, soit 760ms correspondant à la durée de chaque train d'impulsions. Enfin, l'entrée d'horloge du FFd 47 est reliée à la sortie 16ms du compteur 39, alors que son entrée reset est branchée à la sortie 1ms du même compteur. Le FFd 47 va donc générer des impulsions courtes de 0,5ms pour autant que son entrée d soit à 1. Cette entrée est reliée à la sortie d'une porte AND 48 à trois entrées. La première entrée est reliée à la sortie du FFd 45, si bien que ces impulsions ne peuvent être générées que pendant la durée de 24 périodes fixée par ce FFd 45. la deuxième entrée de la porte 48 est reliée par l'intermédiaire de la porte OR 49 à la sortie d'un OR exclusif 50 dont une entrée est branchée à la sortie 32ms du compteur 39, et l'autre entrée à la sortie du démultiplexeur 38 sur laquelle est générée l'information série représentative des informations à transmettre aux moyens d'affichage. La figure 5 donne le timing des signaux aux points A, B et C.

La troisième entrée de la porte 48 est reliée aux moyens de traitement 24 et permet de bloquer l'entrée d du FFd 47 à 0, ce qui peut être considéré comme une fonction ENABLE qui permet de suspendre la génération des impulsions courte indépendamment de l'état de la sortie du démultiplexeur 38. La deuxième entrée de la porte 49 est reliée aux moyens de traitement 24 et permet de maintenir l'entrée d du FFD à 1, ce qui peut être considéré comme la fonction inverse permettant de maintenir la génération des impulsions courtes indépendamment de l'état de sortie du démultiplexeur 38. Nous verrons plus loin l'utilité de ces fonctions ENABL, et ENABLE INVERSE après la description de la figure 5. Les sorties des FFd 44 et 47 sont reliées aux entrées d'une porte OR 51 à la sortie de laquelle apparaît le train d'impulsions courtes formant l'information complète à transmettre au dispositif d'affichage. La sortie de cette porte va à une entrée d'une porte AND 52 dont la deuxième entrée est branchée à la sortie d'un générateur de fréquence 65 kHz 53. Le signal à la sortie de la porte 52 est donc une impulsion de 0,5ms modulée tout ou rien à la fréquence de 65 kHz et constitue le signal radio basse fréquence qui est transmis vers l'extérieur par l'intermédiaire d'un transistor d'amplification 54 et de la bobine émettrice 55.

Enfin la sortie du sélecteur 42 est reliée à un compteur par 4 56 dont les sorties Q1 et Q2 sont reliées au sélecteur de données 34, de manière à sélectionner à tour de rôle les paramètres 25, 26, 27 et 28.

La figure 5 représente la forme caractéristique des signaux à certains points du schéma de la figure 4.

Ainsi on retrouve les sorties 8ms, 16ms et 32ms du compteur 39. La sortie A correspond à l'impulsion de début de cycle à la sortie du FFd 44. B1 et C1 donne la configuration des signaux sur les points B et C lorsque la sortie du démultiplexeur 38 est à 1. On voit que les impulsions courtes correspondantes arrivent pendant que le signal 32ms est à 0. B0 et C0 donne la configuration des signaux sur les points B et C lorsque la sortie du démultiplexeur 38 est à 0. On voit que les impulsions courtes correspondantes arrivent pendant que le signal 32ms est à 1. Ainsi la répartition des impulsions courtes à l'intérieur du train d'impulsions généré à la sortie de la porte 51 est directement représentative de la suite de 0 et de 1 des informations à transmettre aux moyens d'affichage.

On peut constater que, pendant le train d'impulsions, il y a normalement une impulsion courte par période de 32ms, et il a 24 impulsions courtes par train d'impulsions. Or, en utilisant la fonction ENABLE mentionnée à la figure 4, on peut supprimer des impulsions si celles-ci ne servent à rien. Cette suppression d'impulsions peut être utilisée par exemple si une partie des informations n'est pas nécessaire. Par exemple, si la valeur du paramètre à afficher ne comporte que deux chiffres, on peut supprimer les impulsions correspondant au troisième chiffre et éteindre ce dernier plutôt que d'afficher un 0. De même si le dispositif de traitement ne concerne qu'un paramètre, les impulsions concernant le mode peuvent être supprimées, de même que l'affichage du mode, ainsi de suite. Cette combinaison, en permettant de réduire le nombre d'impulsions par train d'impulsions, permet de simplifier le système tout en réduisant la consommation et d'augmenter l'autonomie du dispositif de traitement.

Inversement au moyen de la fonction ENABLE inverse, on peut générer deux impulsions courtes par période de 32ms, ce qui peut être interprété comme un cas particulier, par exemple si la valeur du paramètre à afficher dépasse la capacité d'affichage.

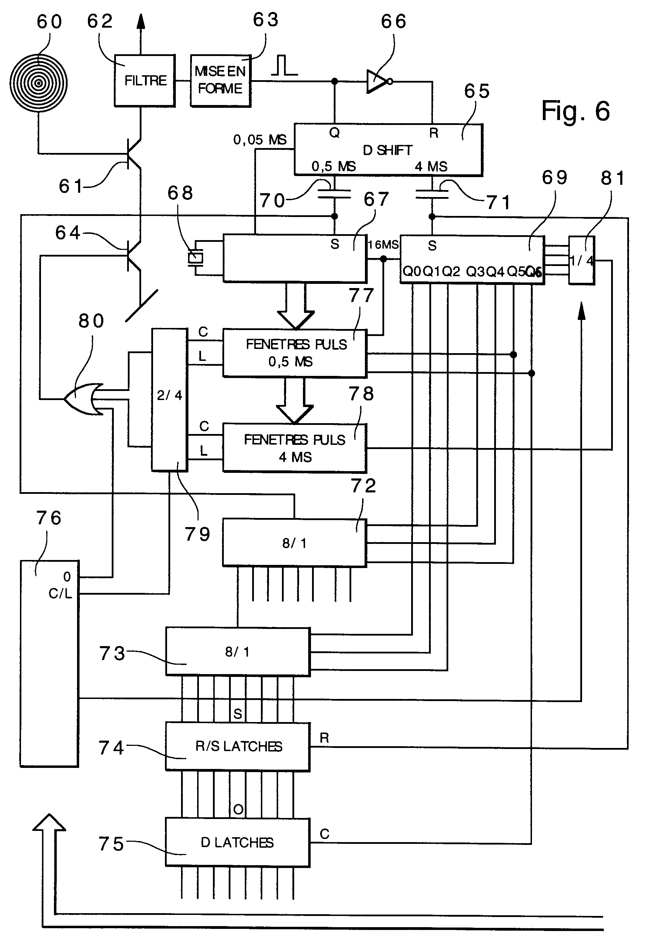

La figure 6 représente à titre d'exemple le schéma bloc des moyens de décodage du dispositif d'affichage permettant de restituer les informations envoyées par le dispositif de traitement. Une bobine de réception 60 est reliée à un amplificateur à transistor 61 dont le collecteur est branché à un filtre 62 relié à un circuit de mise en forme 63. Cette combinaison de circuits permet, lorsque la bobine réceptrice se trouve dans le champ d'une bobine émettrice, de restituer les trains d'impulsions émises par cette dernière. L'émetteur du transistor 61 est relié à un transistor de commutation 64 permettant de mettre hors service l'amplificateur d'entrée. Les trains d'impulsions à la sortie du circuit de mise en forme 63 sont appliqués à l'entrée D d'un schift D 65, et par l'intermédiaire d'un inverseur 66 à l'entrée reset de ce schift 65. L'entrée d'horloge de ce schift est reliée à une sortie d'un générateur de séquences comportant un compteur binaire 67 ayant une période de sortie de 16ms, piloté par un quartz 68 et relié à un deuxième compteur binaire 69. Cette configuration n'est pas sans rappeler le générateur de séquences de la figure 4, et va effectivement servir à restituer les informations émises par le dispositif de traitement. Le schift 65 est agencé de manière à trier les impulsions d'entrée selon leur durée. En l'absence d'impulsions, ce schift est maintenu à 0. Lorsqu'une impulsion apparaît, les différentes étapes du schift basculent à tour de rôle tant que l'impulsion est maintenue. Lorsque l'impulsion dure 0,5ms ou plus, la sortie 0,5ms passe à 1. Cette sortie est reliée par une capa 70 à une entrée de synchronisation du compteur 67 de manière à synchroniser la sortie de ce compteur en phase avec les impulsions courte de 0,5ms représentatives de la suite de 0 et de 1 des informations émises par le dispositif de traitement. Si l'impulsion dure 4ms ou plus, la sortie 4ms du schift 65 passe à 1. Cette sortie est reliée par une capa 71 à une entrée de synchronisation du compteur 69 de manière à synchroniser la sortie de ce compteur en phase avec le cycle de trains d'impulsions émis par le dispositif de traitement. Ainsi, lorsqu'on reçoit correctement ces trains d'impulsions, on peut dire que le générateur de séquences du dispositif d'affichage fonctionne de manière synchrone avec le générateur de séquences du dispositif de traitement et il est ainsi possible de trier les impulsions courtes en fonction de leur répartition dans le temps. Notons que, une fois que la synchronisation est établie, celle-ci est maintenue même pendant une disparition momentanée de la liaison radio en raison de la précision des quartz qui pilotent ces deux générateurs.

Afin de trier les impulsions courtes, celle-ci sont branchées à l'entrée d'un démultiplexeur 8/1 72 dont les entrées de sélection sont reliées aux sorties Q3, Q4 et Q5 du compteur 69. Le démultiplexeur 72 permet de couper le train d'impulsions en six tranches de huit impulsions. Pourquoi des tranches de huit impulsions alors que le train d'impulsions est formé normalement de 24 impulsions représentant six groupes de quatre bits ? Il faut se rappeler que chaque bit offre deux possibilités d'impulsions, l'une correspondant à l'état 0 et l'autre à l'état 1. Ainsi quand on parle de tranches de huit impulsions, on parle de huit impulsions potentiellement possible. Dans le cas d'une information binaire normale, on aura en réalité que quatre impulsions puisqu'on ne peut pas avoir simultanément un 0 et un 1 pour un même bit. Cependant, en utilisant la fonction ENABLE de la figure 4, le nombre d'impulsions peut tomber à 0, ou passer à huit par tranches si on utilise la fonction ENABLE INVERSE.

Pour faciliter l'explication, nous allons décrire la façon de trier une de ces tranches de huit impulsions potentielles, étant entendu que la même disposition se répète six fois, une fois pour chaque tranche. La première sortie du démultiplexeur 72 est reliée à l'entrée d'un deuxième démultiplexeur 8/1 73 dont les entrées de sélection sont reliées aux sorties Q0, Q1 et Q2 du compteur 69. Ainsi chaque tranche de huit impulsions potentielles est divisée en huit tranches de temps, ce qui permet de diriger chaque impulsion vers une sortie différente en fonction de son ordre d'arrivée. Les huit sorties du démultiplexeur 73 sont reliées aux huit entrées set d'un groupe de R/S latches 74 dont les sorties sont reliées aux huit entrées D d'un groupe de D Latches 75. En début de cycle de transmission, les R/S latches 74 sont remis à 0 par l'impulsion de synchronisation délivrée par la capa 71, reliée à l'entrée reset de ces latches. Ces R/S latches vont ensuite passer à 1 pour autant qu'une impulsion apparaisse dans la tranche de temps qui leur est réservée. A la fin du train d'impulsion, la répartition des R/S latches qui sont restés à 0 et de ceux qui sont passés à 1 restituent les informations qui ont été transmises par le dispositif de traitement. L'état des R/S latches est mémorisé en fin de cycle de transmission dans les d latches. A cette fin, l'entrée d'horloge de ces D latches est reliée à la sortie Q6 du compteur 69. Les informations à la sortie des six groupes de D latches (dont un seul est représenté) sont transmises à des moyens de traitement de ces informations 76, moyens agencés de manière à gérer l'affichage de tout ou partie de ces informations, comme à gérer d'autres fonctions, par exemple modifier la durée du cycle de réception en fonction de l'information "période" reçue du dispositif de traitement. A cette fin, une sortie de ces moyens 76 est reliée aux entrées de commande d'un sélecteur permettant de choisir une des quatre périodes disponibles 2, 4, 8 ou 16s.

Enfin, un des problèmes cruciaux, particulièrement au niveau du dispositif d'affichage, est la consommation d'énergie qui conditionne l'autonomie du dispositif. Or l'un des éléments qui consomme le plus d'énergie est l'amplificateur d'entrée, puisque, pour avoir une bonne sensibilité, il faut le polariser en classe A. Cependant, on a besoin de cet amplificateur que pendant un très court instant au moment où une impulsion devrait apparaître, pour voir si cette impulsion est présente ou non. Il est possible au moyen du générateur séquentiel de générer des fenêtres de courte durée synchrones avec les impulsions à recevoir et de ne mettre en service l'amplificateur d'entrée que pendant la durée de ces fenêtres. Un premier circuit 77 relié à des sorties des compteurs 67 et 69 permet de générer des fenêtres synchrones avec les impulsions courtes de 0,5ms. Ce circuit 77 dispose de deux sorties, l'une correspondant à des fenêtres courtes C et l'autre à des fenêtres plus longues L. Un deuxième circuit 78 relié à la sortie du sélecteur de périodes 81 permet de générer des fenêtres synchrones avec les impulsions longues de 4ms de début de cycle de transmission. Ce circuit 78 dispose de deux sorties, l'une correspondant à des fenêtres courtes C et l'autre à des fenêtres plus longues L. Les sorties C et L des circuits 77 et 78 sont branchées aux entrées d'un sélecteur 2/4 permettant de sélectionner soit les fenêtres courtes soit les fenêtres longues, sur commande des moyens de traitement 76. Ces moyens de traitement 76 permettent également de maintenir sur commande l'amplificateur d'entrée en fonctionnement permanent. A cette fin, les moyens de traitement 76 sont reliés à une entrée d'une porte OR 80 qui reçoit sur ses deux autres entrées les signaux correspondant aux fenêtres générées par les circuits 77 et 78. La sortie de la porte 79 commande l'interrupteur à transistor 64 et permet, sur commande des moyens de traitement des informations, de mettre l'amplificateur d'entrée en service en permanence ou pendant des fenêtres courtes ou longues.

## Revendications

1. Système de mesure et d'affichage d'au moins un paramètre essentiellement physiologique au moyen d'au moins un capteur (1, 2, 18, 20, 21) porté par un individu, ledit capteur délivrant des signaux représentatifs dudit paramètre, comprenant d'une part un dispositif autonome de traitement (5) solidaire dudit capteur et pourvu de moyens (24) de mesure, de stockage, d'identification et de codage desdits signaux, lesdits moyens de codage étant agencés de manière à générer aux bornes d'une bobine émettrice (9, 55) des séquences binaires de signaux radio, une desdites séquences constituant une adresse permettant une identification du dispositif de traitement émetteur, les autres séquences étant représentatives de chaque catégorie de mesure et de leur résultat, et d'autre part un dispositif d'affichage (10) séparé et relié au dispositif de traitement par des moyens de communication sans fil, ledit dispositif séparé d'affichage (10) comportant une bobine réceptrice (16, 60), des moyens d'amplification et de mise en forme (63) des signaux aux bornes de ladite bobine réceptrice (16, 60) et des moyens de décodage de ces signaux, caractérisé en ce que les moyens de décodage du dispositif d'affichage (10) comportent un générateur de séquences (77, 78, 79) agencé de manière à ouvrir des fenêtres de détection synchrones avec les trains d'impulsions émis par le dispositif de traitement (24), et à restituer la suite binaire de 1 et de 0 représentative des informations transmise en fonction de la répartition desdites impulsions dans lesdites fenêtres de détection et en ce que les informations transmises par ledit dispositif de traitement (24) sont restituées et affichées seulement si une adresse déterminée a préalablement été identifiée.

2. Système selon la revendication 1, caractérisé par le fait que les moyens d'amplification (61) des signaux aux bornes de la bobine réceptrice (60) sont agencés de manière à n'être mis en service que pendant la durée desdites fenêtres de détection, de manière à réduire la consommation d'énergie.

3. Système selon la revendication 1, caractérisé par le fait que lesdits moyens d'identification (32) sont agencés de manière à fixer cette adresse de manière aléatoire à la mise en service du dispositif.

4. Système selon la revendication 1, caractérisé par le fait que les moyens de décodage du dispositif d'affichage (10) sont agencés de manière à générer une séquence d'initialisation de durée limitée pouvant être enclenchée par une commande externe, cette séquence permettant d'une part de synchroniser le générateur de séquences du dispositif d'affichage (10) sur le générateur de séquences du dispositif de traitement (5) le plus proche, et d'autre part d'enregistrer l'adresse de ce dispositif de traitement (5).

5. Système selon la revendication 1, caractérisé par le fait que les moyens de décodage du dispositif d'affichage (10) sont agencés de manière à générer une séquence de réinitialisation de durée limitée lorsque la réception des signaux devient insuffisante, cette séquence permettant de resynchroniser le générateur de séquences du dispositif d'affichage (10) sur le générateur de séquences du dispositif de traitement (5) correspondant à l'adresse préalablement enregistrée.

6. Système selon la revendication 1, caractérisé par le fait que les moyens de mesure (20, 21), de stockage et d'identification du dispositif de traitement (5) sont agencés de manière à mesurer et mémoriser les résultats de mesure de plusieurs paramètres, et à identifier le paramètre concerné sous forme d'un code binaire incorporé par l'intermédiaire des moyens de codage aux séquences de signaux radio aux bornes de la bobine émettrice.

7. Système selon la revendication 6, caractérisé par le fait que les moyens de codage (39, 40) sont agencés de manière à générer de manière cyclique aux bornes de la bobine émettrice (55) des séquences de signaux correspondant successivement à chacun des paramètres mesurés, identifiés par leur code respectif.

8. Système selon la revendication 7, caractérisé par le fait que les moyens d'affichage (10) comportent des moyens de sélection du paramètre à afficher en réponse à une commande extérieure, moyens de sélection agencés de manière à se synchroniser sur la séquence de signaux radio comportant le code correspondant au paramètre sélectionné.

9. Système selon la revendication 1, caractérisé par le fait que les moyens de mesure du dispositif de traitement (5) sont agencés de manière à fixer la période des séquences de signaux radio générés par les moyens de codage (39, 40), la valeur de cette période étant représentés sous forme d'un code binaire incorporé auxdites séquences de signaux radio.

10. Système selon la revendication 9, caractérisé par le fait que le générateur de séquences des moyens de décodage du dispositif d'affichage (10) est agencé de manière à adapter la période de détection en fonction du code binaire correspondant à cette période émis par les moyens de traitement (24).

11. Système selon la revendication 1, caractérisé par le fait que le dispositif de traitement (5) comporte des moyens pour générer des ordres spéciaux à l'adresse des moyens d'affichage (10) sous forme de codes binaires incorporée par l'intermédiaire des moyens de codage aux séquences de signaux radio aux bornes de la bobine émettrice (55).

12. Système selon la revendication 1, caractérisé par le fait que le dispositif d'affichage (10) est incorporé dans un système plus général utilisant les informations transmises par le dispositif de traitement (5).

13. Système selon la revendication 1, caractérisé en ce qu'au moins un autre capteur permet de délivrer des signaux représentatifs d'un paramètre non physiologique.

14. Système selon la revendication 13, caractérisé en ce que les paramètres non physiologiques sont choisis parmi la vitesse et la puissance développée par l'usager.

## Claims

1. System for measuring and displaying at least one physiological parameter by means of at least one sensor (1, 2, 18, 20, 21) worn by an individual, said sensor providing signals representative of said parameter, comprising on the one hand an autonomous processing device (5) which is attached to said sensor and provided with means (24) for measuring, storing, identifying and coding said signals, said coding means being arranged so as to generate binary sequences of radio signals at the terminals of a transmitting coil (9, 55), one of said sequences constituting an address enabling the transmitting processing device to be identified, the other sequences being representative of each category of measurement and its result, and, on the other hand, a separate display device (10) connected to the processing device by wireless communicating means, said separate display device (10) comprising a receiving coil (16, 60), signal amplifying and shaping means (63) at the terminals of said receiving coil (16, 60) and means for decoding these signals, characterized in that the display device (10) decoding means comprise a sequence generator (77, 78, 79) arranged so as to open detecting windows synchronous with the pulse trains transmitted by the processing device (24), and to restore the binary sequence 0 and 1 representative of the transmitted data as a function of the distribution of said pulses in said detecting windows and in that the data transmitted by said processing device (24) are restored and displayed only if a determined address has previously been identified.

2. System according to claim 1, characterized in that the signal amplifying means (61) at the terminals of the receiving coil (60) are arranged so as to be only set into operation during the course of said detecting windows, so as to reduce the energy consumption.

3. System according to claim 1 characterized in that said identifying means (32) are arranged so as to determine said address randomly when the device is switched on.

4. System according to claim 1, characterized in that the display device (10) decoding means are arranged so as to generate an initialisation sequence of limited duration able to be locked in by an external command, this sequence enabling on the one hand the display device (10) sequence generator to be synchronised with the closest processing device (5) sequence generator, and on the other hand to store the address of this processing device (5).

5. System according to claim 1, characterized in that the display device (10) decoding means are arranged so as to generate a reinitialisation sequence of limited duration when the signal reception becomes insufficient, this sequence enabling the display device (10) sequence generator to be resynchronised with the processing device (5) sequence generator corresponding to the previously stored address.

6. System according to claim 1, characterized in that the processing device (5) measuring (20, 21), storing and identifying means are arranged so as to measure and store the results of several measured parameters, and to identify the parameter concerned in the form of a binary code incorporated via coding means in the radio signal sequences at the terminals of the transmitting coil.

7. System according to claim 6, characterized in that the coding means (39, 40) are arranged so as to generate in a cyclical manner at the terminals of the transmitting coil (55) sequences of signals corresponding successively to each of the measured parameters, identified by their respective codes.

8. System according to claim 7, characterized in that the display means (10) comprise means for selecting the parameter to be displayed in response to an external command, said selecting means being arranged so as to synchronise with the radio signal sequence comprising the code corresponding to the selected parameter.

9. System according to claim 1, characterized in that the processing device (5) measuring means are arranged so as to fix the period of the radio signal sequences generated by the coding means (39, 40), the value of this period being represented in the form of a binary code incorporated in said radio signal sequences.

10. System according to claim 9, characterized in that the display device (10) decoding means sequence generator is arranged so as to adapt the detecting period as a function of the binary code corresponding to this period transmitted by the processing means (26).

11. System according to claim 1, characterized in that the processing device (5) comprises means for generating special orders at the address of the display means (10) in the form of binary codes incorporated via coding means in the radio signal sequences at the terminals of the transmitting coil (55).

12. System according to claim 1, characterized in that the display device (10) is incorporated in a more general system using the data transmitted by the processing device (5).

13. System according to claim 1, characterized in that at least one other sensor enables signals representative of a non physiological parameter to be supplied.

14. System according to claim 13, characterized in that the non physiological parameter are selected from among the speed or the power developed by the individual.

## Patentansprüche

1. Meß- und Anzeigesystem mindestens eines im wesentlichen physiologischen Parameters mittels mindestens eines von einer Person getragenen Sensors (1, 2, 18, 20, 21), der für den Parameter repräsentative Signale liefert, umfassend einerseits eine mit dem Sensor verbundene und mit Meß-, Speicher-, Identifikations- und Kodiermitteln (24) der Signale ausgestattete autonome Verarbeitungseinrichtung (5), welche Kodiermittel derart ausgebildet sind, daß an den Klemmen einer Sendespule (9, 55) binäre Funksignalsequenzen erzeugt werden, von denen eine eine Adresse bildet, die eine Identifikation der sendenden Verarbeitungseinrichtung ermöglicht, während die anderen Sequenzen für jede Meßkategorie und ihr Resultat repräsentativ sind, und andererseits eine von der Verarbeitungseinrichtung separate und mit ihr über drahtlose Mittel verbundene Anzeigeeinrichtung (10), die eine Empfangsspule (16, 60), Verstärkungs- und Formungsmittel (63) von Signalen an den Klemmen der Empfangsspule (16, 60) und Dekodiermittel dieser Signale umfaßt, dadurch gekennzeichnet, daß die Dekodiermittel der Anzeigeeinrichtung (10) einen Sequenzgenerator (77, 78, 79) umfassen, der so ausgebildet ist, daß er Erfassungsfenster synchron mit den von der Verarbeitungseinrichtung (24) abgegebenen Impulsfolgen öffnet und die binäre Folge von 1 und 0 wiederherstellt, die für Informationen repräsentativ ist, die in Abhängigkeit von der Verteilung der Impulse in den Erfassungsfenstern übertragen werden, und daß die von der Verarbeitungseinrichtung (24) übertragenen Informationen nur dann wiederhergestellt und angezeigt werden, wenn vorab eine bestimmte Adresse identifiziert worden ist.

2. System nach Anspruch 1, dadurch gekennzeichnet, daß die Verstärkungsmittel (61) der an den Klemmen der Anfangsspule (60) anstehenden Signale derart ausgebildet sind, daß sie nur während der Dauer der Erfassungsfenster in Betrieb genommen werden, derart, daß der Energieverbrauch reduziert wird.

3. System nach Anspruch 1, dadurch gekennzeichnet, daß die Identifikationsmittel (32) derart ausgebildet sind, daß sie diese Adresse in aleatorischer Weise mit der Inbetriebnahme der Vorrichtung fixieren.

4. System nach Anspruch 1, dadurch gekennzeichnet, daß die Dekodiermittel der Anzeigeeinrichtung (10) derart ausgebildet sind, daß sie eine Initialisationssequenz begrenzter Dauer erzeugen, die von einem externen Befehl auslösbar ist, welche Sequenz einerseits das Synchronisieren des Sequenzgenerators der Anzeigeeinrichtung (10) mit dem Sequenzgenerator der Verarbeitungseinrichtung (5) so nahe wie möglich ermöglicht und andererseits das Registrieren der Adresse dieser Verarbeitungseinrichtung (5).

5. System nach Anspruch 1, dadurch gekennzeichnet, daß die Dekodiermittel der Anzeigeeinrichtung (10) derart ausgebildet sind, daß sie eine Reinitialisationssequenz begrenzter Dauer erzeugen, wenn der Empfang der Signale unzureichend wird, welche Sequenz die Resynchronisation des Sequenzgenerators der Anzeigeeinrichtung (10) auf den Sequenzgenerator der Verarbeitungseinrichtung (5) entsprechend der vorher registrierten Adresse ermöglicht.

6. System nach Anspruch 1, dadurch gekennzeichnet, daß die Meßmittel (20, 21), die Speichermittel und die Identifikationsmittel der Verarbeitungseinrichtung (5) derart ausgebildet sind, daß sie die Meßresultate mehrerer Parameter messen und abspeichern und den betreffenden Parameter in Form eines Binärcodes identifizieren, der über die Kodiermittel in die Funksignalsequenzen an den Klemmen der Sendespule eingefügt wird.

7. System nach Anspruch 6, dadurch gekennzeichnet, daß die Kodiermittel (39, 40) derart ausgebildet sind, daß sie in zyklischer Weise an den Klemmen der Sendespule (55) Signalsequenzen erzeugen, die nacheinander jedem der gemessenen, durch ihren entsprechenden Code identifizierbarten Parameter entsprechen.

8. System nach Anspruch 7, dadurch gekennzeichnet, daß die Anzeigemittel (10) Mittel zum Auswählen des anzuzeigenden Parameters in Reaktion auf einen externen Befehl umfassen, welche Wählmittel derart ausgebildet sind, daß sie sich auf die Funksignalsequenz synchronisieren, die den Code entsprechend dem ausgewählten Parameter umfaßt.

9. System nach Anspruch 1, dadurch gekennzeichnet, daß die Meßmittel der Verarbeitungseinrichtung (5) derart ausgebildet sind, daß sie die Periode der Funksignalsequenzen festlegen, die von den Kodiermitteln (39, 40) erzeugt werden, wobei der Wert dieser Periode in Form eines in die Funksignalsequenzen eingefügten Binärcodes repräsentiert wird.

10. System nach Anspruch 9, dadurch gekennzeichnet, daß der Sequenzgenerator der Dekodiermittel der Anzeigeeinrichtung (10) derart ausgebildet ist, daß er die Erfassungsperiode in Abhängigkeit von dem entsprechenden Binärcode dieser Periode anpaßt, ausgesandt von den Verarbeitungsmitteln (24).

11. System nach Anspruch 1, dadurch gekennzeichnet, daß die Verarbeitungseinrichtung (5) Mittel zum Erzeugen von speziellen Reihenfolgen in der Adresse der Anzeigemittel (10) in Form von Binärcodes umfaßt, die über die Kodiermittel in die Funksignalsequenzen an den Klemmen der Sendespule (55) eingefügt sind.

12. System nach Anspruch 1, dadurch gekennzeichnet, daß die Anzeigeeinrichtung (10) in ein allgemeineres System eingefügt ist, welches von der Verarbeitungseinrichtung (5) übertragene Informationen verwertet.

13. System nach Anspruch 1, dadurch gekennzeichnet, daß mindestens ein weiterer Sensor die Abgabe von Signalen, die für einen nicht physiologischen Parameter repräsentativ sind, ermöglicht.

14. System nach Anspruch 13, dadurch gekennzeichnet, daß die nicht physiologischen Parameter unter der Geschwindigkeit und der von dem Benutzer entwickelten Leistung gewählt sind.
